(19) Europäisches Patentamt
European Patent Office
Office européen des brevets

(11) Veröffentlichungsnummer: **0 374 705 B1**

(12) **EUROPÄISCHE PATENTSCHRIFT**

(45) Veröffentlichungstag der Patentschrift: **21.09.94**

(51) Int. Cl.⁵: **C07D 219/02**, G03F 7/031, C07D 215/14, C07D 221/16

(21) Anmeldenummer: **89122996.5**

(22) Anmeldetag: **13.12.89**

# (54) Photopolymerisierbare Verbindungen, diese enthaltendes photopolymerisierbares Gemisch und daraus hergestelltes photopolymerisierbares Aufzeichnungsmaterial.

(30) Priorität: **22.12.88 DE 3843205**

(43) Veröffentlichungstag der Anmeldung:
**27.06.90 Patentblatt 90/26**

(45) Bekanntmachung des Hinweises auf die Patenterteilung:
**21.09.94 Patentblatt 94/38**

(84) Benannte Vertragsstaaten:
**DE FR GB**

(56) Entgegenhaltungen:
**EP-A- 0 011 786**
**EP-A- 0 167 963**
**DE-A- 2 027 467**
**US-A- 4 444 868**
**US-A- 4 587 200**

**JOURNAL OF POIYMER SCIENCE, PART A, POLYMER CHEMISTRY, Band 25, Nr. 11,November 1987, Seiten 3063-3077, John Wiley and Sons, New York, US; K. ICHIMURAet al.: "Photosensitive poly(methaacrylates) having styrylpyridinium andstyrylquinolinium groups"**

(73) Patentinhaber: **HOECHST AKTIENGESELLSCHAFT**

**D-65926 Frankfurt (DE)**

(72) Erfinder: **Steppan, Hartmut, Dr.**
**Panoramastrasse 17**
**D-6200 Wiesbaden (DE)**
Erfinder: **Frommeld, Hans-Dieter, Dr.**
**Simrockstrasse 7A**
**D-6200 Wiesbaden (DE)**

EP 0 374 705 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue photopolymerisierbare Verbindungen, ein photopolmerisierbares Gemisch, das diese Verbindungen enthält, sowie ein photopolymerisierbares Aufzeichnungsmaterial aus einem Schichtträger und einer photopolymerisierbaren Schicht, die aus dem Gemisch besteht.

Aus der DE-C 20 27 467 sind photopolymerisierbare Gemische bekannt, die polymere Bindemittel, polymerisierbare Verbindungen und als Photoinitiatoren Derivate des Acridins und Phenazins enthalten. Einige Vertreter dieser Verbindungsklasse, z. B. das 9-Phenyl-acridin, zeichnen sich durch eine hohe Lichtempfindlichkeit aus. Die bevorzugten Vertreter haben den Nachteil, daß sie dazu neigen, aus photopolymerisierbaren Schichten und insbesondere aus lichtgehärteten Photoresistschichten in bestimmte Behandlungsbäder, z. B. saure galvanische Bäder zu wandern und dort eine störende Gelbfärbung hervorzurufen.

Aus den EP-A 11 786 und 220 589 sind entsprechende Gemische bekannt, die als Photoinitiatoren bestimmte Chinolinderivate enthalten. Auch diese Gemische, die ähnlich lichtempfindlich wie die vorstehend genannten sind, leiden unter den gleichen Nachteilen.

In der US-A 4 444 868 wird ein lichtempfindliches Gemisch beschrieben, das lichtvernetzbare polymere Verbindungen mit seitenständigen Chinolinylvinylphenylgruppen enthält.

In J. Pol. Sci. A, Polymer Chemistry, Bd. 25, S. 3063-3077 (1987) werden ähnliche Polymere beschrieben, die durch Polymerisation von Styrylpyridinium- oder Styrylchinoliniumgruppen enthaltenden (Meth)-acrylaten erhalten werden.

Die Lichtreaktion dieser bekannten Polymeren beruht auf einer Lichtvernetzung der seitenständigen ungesättigten Substituenten.

In der EP-A 167 963 sind lichtempfindliche Gemische beschrieben, die Diazoniumsalz-Polykondensationsprodukte, radikalisch polymerisierbare Verbindungen, Photopolymerisationsinitiatoren und Bindemittel enthalten. Als Photoinitiatoren werden u.a. in 9-Stellung substituierte Acridine, z.B. das 9-Acryloylamino-acridin eingesetzt.

Die bekannten photopolymerisierbaren Materialien haben häufig im lichtgehärteten Zustand eine für manche Zwecke nicht ausreichende mechanische Festigkeit und Härte. Dies gilt besonders bei Anwendungen als Lötstopmasken, als Permanentresist und als Flachdruckplatten.

Aufgabe der Erfindung war es, photopolymerisierbare Verbindungen und Gemische zur Verfügung zu stellen, die Aufzeichnungsmaterialien mit gleich guter Lichtempfindlichkeit und Bildwiedergabe wie die bevorzugten bekannten Gemische ergeben, wobei die Photoinitiatoren aber eine geringere Wanderungstendenz aus der photopolymerisierbaren oder photopolymerisierten Schicht zeigen und die lichtgehärteten Schichten eine höhere mechanische Resistenz und Festigkeit aufweisen.

Erfindungsgemäß werden Verbindungen der allgemeinen Formel I

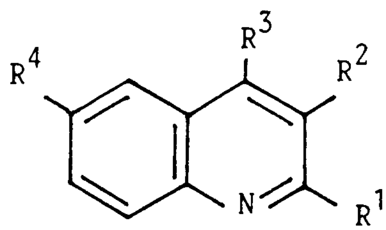

(I)

vorgeschlagen, worin

$R^1$ und $R^2$ gemeinsam einen ggf. substituierten 5- oder 6-gliedrigen carbocyclischen Ring bilden,

$R^3$ eine Phenylgruppe, die einen oder mehrere Substituenten X trägt,

$R^4$ ein Wasserstoff- oder Halogenatom, eine Methylgruppe, eine ggf. substituierte Benzoylgruppe oder eine Gruppe der Formel

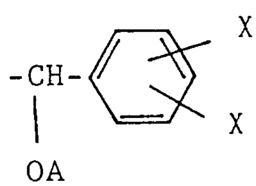

,

n Null oder 1,

X ein Wasserstoff- oder Chloratom, eine Alkylgruppe mit 1 bis 4 Kohlenstoffatomen oder eine der Gruppen OA, $CH_2OA$, $CH_2NHA$ oder $C_2H_4OA$ bedeutet, wobei mehrere X untereinander gleich oder verschieden sein können, und

A eine Acryloyl- oder Methacryloylgruppe ist,

wobei die Verbindungen jeweils mindestens eine Gruppe A enthalten.

Erfindungsgemäß wird ferner ein photopolymerisierbares Gemisch vorgeschlagen, das als wesentliche Bestandteile

a) ein polymeres Bindemittel,

b) eine polymerisierbare Verbindung mit mindestens einer endständigen ethylenischen Doppelbindung und einem Siedepunkt oberhalb 100 ° C bei Normaldruck und

c) eine N-heterocyclische Verbindung als Photoinitiator

enthält.

Das erfindungsgemäße Gemisch ist dadurch gekennzeichnet, daß die N-heterocyclische Verbindung eine Verbindung der vorstehenden allgemeinen Formel I ist.

Erfindungsgemäß wird schließlich ein photopolymerisierbares Aufzeichnungsmaterial mit einem Schichtträger und einer darauf aufgebrachten photopolymerisierbaren Schicht vorgeschlagen, die aus dem vorstehend definierten photopolvmerisierbaren Gemisch besteht.

Die erfindungsgemäßen Verbindungen sind neu. Sie werden durch Verestern der ihnen zugrundeliegenden Hydroxyverbindungen mit aktivierten Derivaten, z. B. Anhydriden oder Chloriden, der Acryl- oder Methacrylsäure hergestellt. Die Hydroxyverbindungen sind teils bekannt, teils werden sie in Analogie zu bekannten Verfahren hergestellt. Das 9-(4-Hydroxyphenyl)acridin ist bekannt.

Verbindungen der Formel I, in denen $R^1$ und $R^2$ einen Benzolring bilden, werden hergestellt, indem Diphenylamin oder seine einfachen Substitutionsprodukte, z. B. 3-Methyldiphenylamin oder ein Chlordiphenylamin, mit Benzoesäure oder einfachen Benzoesäurederivaten, z. B. t-Butylbenzoesäure, Benzophenon-4-carbonsäure, Diphenyl-4-carbonsäure, 4-Aminomethyl-benzoesäure oder Terephthalsäure in einem geeigneten Reaktionsmedium wie Polyphosphorsäure bei etwa 150 - 200 °C zum 2,7-Dibenzoyl-9-phenylacridinderivat kondensiert werden. Anschließend werden die Carbonylgruppen der ggf. substituierten Benzoylgruppen z. B. mit Natriumboranat zur -CHOH-Gruppe reduziert.

Von den erfindungsgemäßen Verbindungen werden diejenigen bevorzugt, in denen $R^1$ und $R^2$ zu einem Ring verbunden sind, der ein Benzolring ist. $R^3$ ist eine Phenylgruppe, die insbesondere einen oder mehrere Substituenten X trägt. Ein Substituent steht bevorzugt in 4-Stellung. Wenn $R^1$ und $R^2$ zusammen einen Benzolring bedeuten, ist dieser vorzugsweise substituiert, insbesondere in der 2-Stellung des mit ihm gebildeten Acridinkerns. Als Substituenten kommen vor allem solche der Bedeutung von $R^4$ in Betracht. Acridinverbindungen, in denen die 2- und 7-Stellung solche Substituenten trägt, sind besonders vorteilhaft, da sie ein stark reduziertes Diffusionsvermögen aufweisen, analog den in der gleichzeitig eingereichten EP-A 337.704 beschriebenen Verbindungen. Die erfindungsgemäßen Verbindungen können eine oder mehrere, im allgemeinen 1 bis 3, Acryloyl- oder Methacryloylgruppen aufweisen, wobei Verbindungen mit zwei polymerisierbaren Gruppen wegen ihrer Vernetzungswirkung besonders vorteilhaft sind.

Beispiele für geeignete erfindungsgemäße Verbindungen sind, 3-(3,4-Bis-acryloyloxy-benzyliden)-9-methyl-2,3-dihydro-1H-cyclopenta[b]chinolin, 3-(2,4-Bis-methacryloyloxy-benzyliden)-7-chlor-9-(2-chlorphenyl)-2,3-dihydro-1H-cyclopenta[b]chinolin, 2,7-Dimethyl-9-(4-acryloylaminomethyl-phenyl)acridin, 2,7-Bis-($\alpha$-methacryloyloxy-4-tert.butyl-benzyl)-9-(4-tert.-butyl-phenyl)acridin, 2,7-Bis-(4-acryloyloxymethylbenzoyl)-9-(4-acryloyloxymethylphenyl)acridin, 9-(4-methacryloyloxyethylphenyl)acridin und 2,7-Bis-($\alpha$-methacryloyloxy-benzyl)-9-phenyl-acridin.

Der Mengenanteil der Verbindungen der Formel I in dem erfindungsgemäßen Gemisch beträgt im allgemeinen 0,01 bis 10, vorzugsweise 0,1 bis 5 Gew.-%, bezogen auf die nichtflüchtigen Bestandteile.

Für die Zwecke der Erfindung geeignete polymerisierbare Verbindungen sind bekannt und z. B. in den US-A 2 760 863 und 3 060 023 beschrieben.

Bevorzugte Beispiele sind Acryl- und Methacrylsäureester von ein- oder mehrwertigen, bevorzugt mindestens zweiwertigen Alkoholen, wie Ethylenglykoldiacrylat, Polyethylenglykoldimethacrylat, Acrylate und Methacrylate von Trimethylolethan, Trimethylolpropan, Pentaerythrit und Dipentaerythrit und von mehrwertigen alicyclischen Alkoholen oder N-substituierte Acryl- und Methacrylsäureamide. Mit Vorteil werden auch Umsetzungsprodukte von Mono- oder Diisocyanaten mit Partialestern mehrwertiger Alkohole eingesetzt. Derartige Monomere sind in den DE-A 20 64 079, 23 61 041 und 28 22 190 beschrieben. Der Mengenanteil der Schicht an Monomeren beträgt im allgemeinen etwa 10 bis 80, vorzugsweise 20 bis 60 Gew.-%.

Das Gemisch enthält außerdem noch ein polymeres Bindemittel. Als Bindemittel kann eine Vielzahl löslicher organischer Polymerisate Einsatz finden.

Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit besonderem Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrigalkalischen Entwicklern entwickeln lassen. Derartige Bindemittel können z B. die folgenden Gruppen enthalten: -COOH, $-PO_3H_2$, $-SO_3H$; $-SO_2NH-$, $-SO_2-NH-SO_2-$ und $-SO_2-NH-CO-$.

Als Beispiele hierfür seien genannt: Maleinatharze, Polymerisate aus N-(p-Tolyl-sulfonyl)-carbaminsäure-$\beta$-(methacryloyloxy)-ethylester und Mischpolymerisate dieser und ähnlicher Monomerer mit anderen Monomeren sowie Vinylacetat/Crotonsäure- und Styrol/Maleinsäureanhydrid-Mischpolymerisate. Alkylmethacrylat/Methacrylsäure-Mischpolymerisate und Mischpolymerisate aus Methacrylsäure, höheren Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:

Inhibitoren zur Verhinderung der thermischen Polymerisation der Monomeren,
Wasserstoffdonatoren,
die spektrale Lichtempfindlichkeit derartiger Schichten modifizierende Stoffe,
Farbstoffe,
gefärbte und ungefärbte Pigmente,
Farbbildner,
Indikatoren,
Weichmacher, z.B. Polyglykole oder Ester der p-Hydroxybenzoesäure.

Diese Bestandteile sind zweckmäßig so auszuwählen, daß sie in dem für den Initiierungsvorgang wichtigen aktinischen Strahlungsbereich möglichst wenig absorbieren.

Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Aufzeichnungsmaterial auf dem Reproduktionsgebiet.

Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt: Aufzeichnungsschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von kopierten Schaltungen und für das Formteilätzen, anwendbar. Besondere Bedeutung haben die erfindungsgemäßen Gemische als Kopierschichten für die photomechanische Herstellung von Flachdruckformen und für die Photoresisttechnik.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung kopierter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen, Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das

sich in der Entwicklerflüssigkeit löst oder mindestens an den nicht gehärteten Stellen bei der Entwicklung entfernen läßt. Hierfür geeignete Materialien sind z B. Wachse, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die Herstellung der lichtempfindlichen Materialien unter Verwendung des erfindungsgemäßen Gemischs erfolgt in bekannter Weise.

So kann man dieses in einem Lösungsmittel aufnehmen und die Lösung bzw. Dispersion durch Gießen, Sprühen, Tauchen, Antrag mit Walzen usw. auf den vorgesehenen Träger als Film antragen und anschließend antrocknen. Dicke Schichten (z. B. von 250 $\mu$m und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und angetrocknet. Die lichtempfindlichen Schichten - Dicke etwa zwischen 10 und 100 $\mu$m - werden dann gleichfalls zunächst durch Laminieren zusammen mit dem temporären Träger auf das gewünschte Substrat auflaminiert.

Die Verarbeitung der Materialien wird in bekannter Weise vorgenommen. Zur Entwicklung werden sie mit einer geeigneten Entwicklerlösung, bevorzugt einer schwach alkalischen wäßrigen Lösung, behandelt, wobei die unbelichteten Anteile der Schicht entfernt werden und die belichteten Bereiche der Kopierschicht auf dem Träger zurückbleiben.

Die erfindungsgemäßen Aufzeichnungsmaterialien zeichnen sich durch einen geringen Lichtempfindlichkeitsverlust beim Lagern aus. Dieser Vorteil wird offenbar durch eine höhere Diffusionsfestigkeit der Initiatoren in der photopolymerisierbaren Schicht gegenüber dem unsubstituierten 9-Phenyl-acridin bewirkt. Aus der lichtgehärteten Schicht wandern die Initiatoren nicht oder in wesentlich geringerem Maße aus als bekannte Initiatoren. Die Materialien ergeben nach der Belichtung Schichten mit höherer mechanischer Festigkeit und Resistenz gegen Abrieb, z. B. beim Druck.

Im folgenden werden Beispiele für das erfindungsgemäße Gemisch angegeben. Dabei wird zunächst die Herstellung von Verbindungen der Formel 1 beschrieben. Anschließend werden Anwendungsbeispiele für die photopolymerisierbaren Gemische beschrieben.

In den Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

## Herstellungsbeispiele

1.) 2,7-Dimethyl-9-(4-acryloylaminomethyl-phenyl)-acridin

197 Gt (1 mol) 4,4'-Dimethyl-diphenylamin und 151 Gt (1 mol) 4-Aminomethyl-benzoesäure wurden in 5000 Gt Polyphosphorsäure 1 Stunde auf 200 °C erhitzt. Nach dem Abkühlen wurde das Reaktionsgemisch mit Wasser und Ammoniak versetzt, das 2,7-Dimethyl-9-(4-aminomethyl-phenyl)-acridin abgesaugt, gereinigt und getrocknet (Fp 220 °C).

Das Produkt wurde mit Acrylsäurechlorid und Triethylamin in Aceton acryliert.

2.) 2,7-Bis($\alpha$-methacryloyloxy-benzyl)-9-phenylacridin

1 Gt Diphenylamin und 15 Gt Polyphosphorsäure wurden unter Rühren auf 100 °C erwärmt. Nach Zugabe von 2,5 Gt Benzoesäure wurde das Gemisch 45 Minuten auf 200 °C erhitzt. Nach dem Abkühlen auf 100 °C wurde die Mischung in 70 Gt Wasser gegossen, das 2,7-Dibenzoyl-9-phenylacridin abfiltriert und gereinigt (Fp 210 °C).

1 Gt 2,7-Dibenzoyl-9-phenylacridin wurde in 4 Gt Ethanol suspendiert und mit 0,1 Gt Natriumboranat (portionsweise) bei 20 - 50 °C reduziert. Nach 24 Stunden wurde das Reaktionsprodukt 2,7-Bis-($\alpha$-hydroxybenzyl)-9-phenylacridin mit Wasser ausgefällt, gereinigt und getrocknet (Fp oberhalb 280 °C).

1 Gt 2,7-Bis-($\alpha$-hydroxybenzyl)-9-phenylacridin wurde in 4 Gt Aceton suspendiert und mit 0,8 Gt Methacrylsäureanhydrid und 0,001 Gt 4-Dimethylamino-pyridin 1 Stunde unter Rückfluß erhitzt. Das Gemisch wurde danach mit Wasser versetzt und das Produkt abfiltriert und getrocknet.

## Anwendungsbeispiele

### Beispiel 1

Die folgenden zwei Beschichtungslösungen wurden hergestellt aus:

52 Gt eines Terpolymerisats aus Styrol, Methacrylsäure und n-Hexylmethacrylat (10:30:60; Säurezahl 190),

17,6 Gt Polyethylenglykol-400-dimethacrylat,
0,04 Gt eines blauen Azofarbstoffs, erhalten durch Kuppeln von 2,4-Dinitro-6-chlor-benzoldiazoniumsalz mit 2-Methoxy-5-acetylamino-N,N-diethylanilin,
0,12 Gt 1,4-Bis-(4-tert.butoxy-phenylamino)-5,8-dihydroxy-anthrachinon,
90 Gt Butanon und
50 Gt Ethanol,
denen als Photoinitiator

a) 1 Gt 2,7-Dimethyl-9-(4-methacryloylaminomethyl-phenyl)-acridin und
b) 1 Gt 2,7-Dimethyl-9-(4-acetylaminomethyl-phenyl)-acridin (Vergleich)

zugesetzt wurde. Mit den angegebenen Lösungen wurde jeweils eine biaxial verstreckte und thermofixierte Polyethylenterephthalatfolie der Stärke 25 µm beschichtet und getrocknet (Schichtdicke jeweils 100 µm). Die so gefertigten Trockenresistfolien wurden anschließend mit einer Polypropylenfolie abgedeckt.

Die Resistschichten wurden nach Abziehen der Polypropylendeckfolie in einer handelsüblichen Laminiervorrichtung bei 115 °C auf eine mit 35 µm starker Kupferfolie kaschierte Phenoplast-Schichtstoffplatte laminiert und 20 Sekunden mit einem handelsüblichen Belichtungsgerät (5 kW Metallhalogenid-Lampe) belichtet. Als Vorlage diente ein 13-stufiger Belichtungskeil mit Dichteinkrementen von 0,15. Anschließend wurden die Platten mit 1 ,0 %iger Sodalösung in einem Sprühprozessor 90 Sekunden entwickelt. Beide Resists waren bis zur Stufe 8 gehärtet.

An vollflächig belichteten Mustern wurde dann die Härte der Schicht bestimmt. Hierzu wurde die Eindruck-Härteprüfung nach Buchholz, beschrieben in DIN 53 153, angewandt. Bei dieser Prüfung wird mittels eines geeigneten Geräts das Eindringen eines Kreismessers in die zu prüfende Schicht unter definierter Belastung gemessen. Dabei wird ein Eindruck erhalten, der umso länger ist, je weicher das zu prüfende Material war. Als Härtezahl wird dann der Wert

$$\frac{100}{\text{Eindrucklänge in mm}}$$

angegeben.

Das erfindungsgemäße Muster hatte die Buchholzhärte 81, das Vergleichsmuster die Härte 72.

Beispiel 2

In Beschichtungslösungen aus
40 Gt eines Copolymerisats aus Methylmethacrylat und Methacrylsäure (Säurezahl 115),
40 Gt Trimethylolpropantriacrylat und
0,5 Gt Farbstoff Disperse Blue 134 (C.I. 61 551) in
520 Gt 2-Methoxy-ethanol
wurden als Photoinitiatoren

a) 1,2 Gt 2,7-Bis-(α-acryloyloxybenzyl)-9-phenylacridin und
b) 1,2 Gt 2,7-Bis-(α-acetoxybenzyl)-9-phenylacridin (Vergleich)

gelöst. Die Lösungen wurden auf elektrolytisch aufgerauhtes und anodisiertes 0,3 mm starkes Aluminium durch Aufschleudern aufgetragen. Die Schicht wurde 2 Minuten bei 100 °C getrocknet, wobei man ein Schichtgewicht von 2,4 g/m² erhielt. Anschließend wurden die Platten mit einer Polyvinylalkohol-Deckschicht von 4 g/m² versehen.

Die so erhaltenen Druckplatten wurden mit einer 5-kW-Metallhalogenidlampe 15 Sekunden in einem Abstand von 110 cm unter einer Negativtestvorlage zusammen mit einem 13-stufigen Belichtungskeil, der Dichteinkremente von 0,15 enthält, belichtet, Die durch Licht nicht gehärteten Teile wurden durch überwischen mit einer Entwicklerlösung folgender Zusammensetzung entfernt:
3 Gt Natriummetasilikat x 9 $H_2O$,
0,05 Gt Strontiumchlorid,
0,03 Gt nichtionogenes Netzmittel (Kokosfettalkohol-Polyoxyethylenether mit ca. 8 Oxyethyleneinheiten)
0,003 Gt Antischaummittel,
100 Gt vollentsalztes Wasser.

Anschließend wurden die Druckplatten nebeneinander in eine Offsetdruckmaschine gespannt und unter forcierten Bedingungen (verdoppelter Anpreßdruck zwischen Gummituch und Druckplatte unter Einsatz einer besonders abrasiven Druckfarbe) getestet. Die Druckergebnisse der Platte 2 b ließen ab 70.000 nach.

Bei der Auflage 80.000 war die Farbannahme der Platte 2 b stark reduziert, die Drucke der Platte 2 a waren dagegen noch erstklassig.

Beispiel 3

Eine Beschichtungslösung aus
40 Gt des in Beispiel 1 angegebenen Terpolymerisats,
40 Gt des Diurethans aus 1 mol 2,2,4-Trimethyl-hexamethylendiisocyanat und 2 mol 2-Hydroxyethylmethacrylat,
0,04 Gt 1,4-Bis-(4-tert.-butoxy-phenylamino)-5,8-dihydroxy-anthrachinon,
0,75 Gt Leukokristallviolett,
0,5 Gt 3-(4-methacryloyloxy-benzyliden-9-phenyl-2,3-dihydro-1H-cyclopenta[b]chinolin und
0,6 Gt 2,2-Dibrommalonsäureamid in
90 Gt Butanon und
50 Gt Ethanol
wurde auf eine 25 µm starke Polyethylenterephthalatfolie aufgebracht. Anschließend wurde 2 Minuten bei 100 °C im Trockenschrank getrocknet. Man erhielt mit einem Schichtgewicht von 44 g/m² einen Trockenresist, der für Ätzverfahren und Galvanoanwendungen besonders gut geeignet ist.

## Patentansprüche

**1.** Verbindungen der allgemeinen Formel I

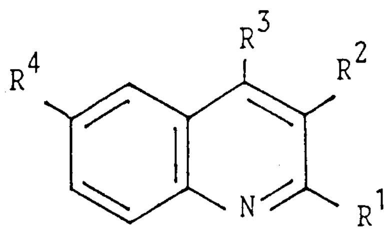

(I)

worin

R¹ und R² gemeinsam einen ggf. substituierten 5- oder 6-gliedrigen carbocyclischen Ring bilden,
R³ eine Phenylgruppe, die einen oder mehrere Substituenten X trägt,
R⁴ ein Wasserstoff- oder Halogenatom, eine Methylgruppe, eine ggf. substituierte Benzoylgruppe oder eine Gruppe der Formel

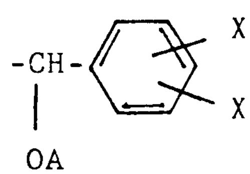

,

n Null oder 1,
X ein Wasserstoff- oder Chloratom, eine Alkylgruppe mit 1 bis 4 Kohlenstoffatomen oder eine der Gruppen OA, $CH_2OA$, $CH_2NHA$ oder $C_2H_4OA$ bedeutet, wobei mehrere X untereinander gleich oder verschieden sein können, und
A eine Acryloyl- oder Methacryloylgruppe ist,

wobei die Verbindungen jeweils mindestens eine Gruppe A enthalten.

**2.** Verbindungen nach Anspruch 1, dadurch gekennzeichnet, daß $R^1$ und $R^2$ gemeinsam einen Benzolring bilden.

**3.** Verbindungen nach Anspruch 1 oder 2, dadurch gekennzeichnet, daß $R^3$ eine Phenylgruppe ist, die mindestens einen Substituenten X trägt.

**4.** Verbindungen nach Anspruch 2, dadurch gekennzeichnet, daß der Benzolring mindestens einen Substituenten der Bedeutung von $R^4$ trägt.

**5.** Verbindungen nach Anspruch 4, dadurch gekennzeichnet, daß der Substituent $R^4$ in der 2-Stellung des mit dem Benzolring gebildeten Acridinkerns steht.

**6.** Photopolymerisierbares Gemisch, das als wesentliche Bestandteile
a) ein polymeres Bindemittel,
b) eine polymerisierbare Verbindung mit mindestens einer endständigen ethylenischen Doppelbindung und einem Siedepunkt oberhalb 100° C bei Normaldruck und
c) eine N-heterocyclische Verbindung als Photoinitiator
enthält, dadurch gekennzeichnet, daß die N-heterocyclische Verbindung eine Verbindung der allgemeinen Formel I gemäß einem der Ansprüche 1 bis 5 ist.

**7.** Photopolymerisierbares Gemisch nach Anspruch 6, dadurch gekennzeichnet, daß das Bindemittel wasserunlöslich und in wäßrig-alkalischen Lösungen löslich ist.

**8.** Photopolymerisierbares Gemisch nach Anspruch 6, dadurch gekennzeichnet, daß die polymerisierbare Verbindung ein Ester der Acryl- oder Methacrylsäure mit einem aliphatischen Alkohol ist.

**9.** Photopolymerisierbares Aufzeichnungsmaterial mit einem Schichtträger und einer photopolymerisierbaren Schicht, dadurch gekennzeichnet, daß die Schicht aus einem Gemisch gemäß einem der Ansprüche 6 bis 8 besteht.

## Claims

**1.** Compounds of the general formula I

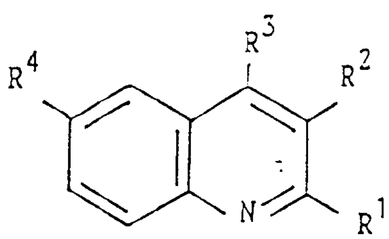

(I)

in which

| | |
|---|---|
| $R^1$ and $R^2$ | jointly form an optionally substituted 5- or 6-membered carbocyclic ring, |
| $R^3$ | is a phenyl group carrying one or several substituents X, |
| $R^4$ | is a hydrogen or halogen atom, a methyl group, an optionally substituted benzoyl group or a group of the formula |

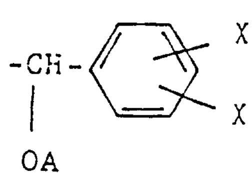

| | |
|---|---|
| n | is zero or 1, |
| X | is a hydrogen or chlorine atom, an alkyl group containing 1 to 4 carbon atoms or one of the groups OA, $CH_2OA$, $CH_2NHA$ or $C_2H_4OA$, where a plurality of Xs may be identical or different from one another, and |
| A | is an acryloyl or methacryloyl group, |

the compounds containing at least one group A in each case.

**2.** Compounds as claimed in claim 1, wherein $R^1$ and $R^2$ jointly form a benzene ring.

3. Compounds as claimed in claim 1 or 2, wherein $R^3$ is a phenyl group which carries at least one substituent X.

4. Compounds as claimed in claim 2, wherein the benzene ring carries at least one substituent having the meaning of $R^4$.

5. Compounds as claimed in claim 4, wherein the substituent $R^4$ is in the 2-position of the acridine nucleus formed with the benzene ring.

6. A photopolymerizable mixture which contains as essential constituents
   a) a polymeric binder
   b) a polymerizable compound having at least one terminal ethylenic double bond and a boiling point above 100 °C at normal pressure, and
   c) a N-heterocyclic compound as photoinitiator,

   wherein the N-heterocyclic compound is a compound of the general formula I as claimed in one of claims 1 to 5.

7. A photopolymerizable mixture as claimed in claim 6, wherein the binder is water-insoluble and soluble in agueous alkaline solutions.

8. A photopolymerizable mixture as claimed in claim 6, wherein the polymerizable compound is an ester of acrylic or methacrylic acid with an aliphatic alcohol.

9. A photopolymerizable recording material comprising a layer support and a photopolymerizable layer, wherein said layer is composed of a mixture as claimed in one of claims 6 to 8.

**Revendications**

1. Composés de formule générale I

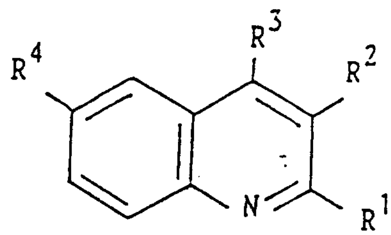

(I)

dans laquelle

| | |
|---|---|
| $R^1$ et $R^2$ | forment ensemble un cycle carbocyclique à 5 ou 6 chaînons, éventuellement substitué, |
| $R^3$ | représente un groupe phényle qui porte un ou plusieurs substituants X, |
| $R^4$ | représente un atome d'hydrogène ou d'halogène, le groupe méthyle, un groupe benzoyle éventuellement substitué ou un groupe de formule |

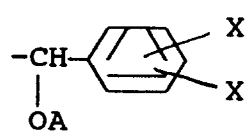

| | |
|---|---|
| n | est zéro ou 1, |
| X | représente un atome d'hydrogène ou de chlore, un groupe alkyle ayant de 1 à 4 atomes de carbone ou l'un des groupes OA, $CH_2OA$, $CH_2NHA$ ou $C_2H_4OA$, plusieurs restes X pouvant être identiques ou différents les uns des autres, et |
| A | est un groupe acryloyle ou méthacryloyle, |

les composés contenant chacun au moins un groupe A.

**2.** Composés selon la revendication 1, caractérisés en ce que $R^1$ et $R^2$ forment ensemble un cycle benzénique.

**3.** Composés selon la revendication 1 ou 2, caractérisés en ce que $R^3$ est un groupe phényle qui porte au moins un substituant X.

**4.** Composés selon la revendication 2, caractérisés en ce que le cycle benzénique porte au moins un substituant ayant la signification de $R^4$.

**5.** Composés selon la revendication 4, caractérisés en ce que le substituant $R^4$ se trouve en position 2 du noyau acridine formé avec le cycle benzénique.

**6.** Composition photopolymérisable, contenant en tant que composants essentiels

a) un liant polymère,

b) un composé polymérisable comportant au moins une double liaison éthylénique en bout de chaîne et ayant un point d'ébullition supérieur à 100 °C sous la pression normale, et

c) un composé N-hétérocyclique en tant que photo-initiateur,

caractérisé en ce que le composé N-hétérocyclique est un composé de formule générale I selon l'une des revendications 1 à 5.

**7.** Composition photopolymérisable selon la revendication 6, caractérisée en ce que le liant est insoluble dans l'eau et soluble dans des solutions aqueuses-alcalines.

**8.** Composition photopolymérisable selon la revendication 6, caractérisée en ce que le composé polymérisable est un ester de l'acide acrylique ou méthacrylique avec un alcool aliphatique.

**9.** Matériau de reproduction photopolymérisable comportant un support de couche et une couche photopolymérisable, caractérisé en ce que la couche est constituée d'une composition selon l'une des revendications 6 à 8.